# EUROPÄISCHE PATENTSCHRIFT

(11) **EP 1 350 030 B1**
(45) Veröffentlichungstag und Bekanntmachung des Hinweises auf die Patenterteilung: **12.10.2005**
(21) Anmeldenummer: 01272039.7
(22) Anmeldetag: 21.12.2001
(51) Int. Cl.: F04B 43/04, A61L 9/12, A01M 1/20

(54) **DOSIERVORRICHTUNG ZUR FÖRDERUNG GERINGER STOFFMENGEN**
DOSING DEVICE FOR CONVEYING SMALL AMOUNTS OF SUBSTANCES
DISPOSITIF DE DOSAGE SERVANT A ACHEMINER DE PETITES QUANTITES DE MATIERE

(30) Priorität: 22.12.2000 DE 10065855
(43) Veröffentlichungstag der Anmeldung: 08.10.2003
(73) Patentinhaber: BSH Bosch und Siemens Hausgeräte GmbH, 81739 München (DE)
(72) Erfinder: FRICKE Christian, Dr., 85221 Dachau (DE)
(86) Internationale Anmeldenummer: PCT/EP2001/015201
(87) Internationale Veröffentlichungsnummer: WO 2002/052154

(56) Entgegenhaltungen:
- WO-A-94/19609
- JP-A- 2 302 534
- US-A- 5 899 381
- ANDERS OLSSON ET AL: "The first valve-less diffuser gas pump" PROC. IEEE 10TH ANN. INT. WORKSHOP ON MICRO ELECTRO MECH. SYSTEMS, 26. - 30. Januar 1997, Seite 108 XP010216888 Nagoya

## Beschreibung

Die Erfindung betrifft eine Dosiervorrichtung gemäß dem Oberbegriff des Anspruchs 1.

Eine solche Dosiervorrichtung ist aus der WO9419609 A bekannt. Innerhalb der Mikrosystemtechnik finden Mikropumpen als spezielle Aktoren in vielen Bereichen Verwendung. Ihre Verwendung erlaubt es, minimale Mengen eines Gases oder einer Flüssigkeit in genau bemessenen Dosen zu fördern. Neben dem Einsatz in der Labortechnik werden Mikropumpen auch in der modernen Bürotechnik eingesetzt, hierbei macht ihre Verwendung in Tintenstrahldruckern einen großen Anteil aus. Diese Mikropumpen zeichnen sich durch eine kompakte Bauweise und hohe Präzision hinsichtlich der Dosierung aus, welche man erreicht, indem man neue Materialien einsetzt und mit dem Pumpelement direkt auf die zu fördernden Stoffe einwirkt.

Vor der gleichen Aufgabe, minimale Stoffmengen freizusetzen, steht man bei der Aromatisierung, wie sie z. B. beim Trockenvorgang in Wäschetrocknern vorgenommen wird. Bei der Zugabe von Duftstoffen werden gegenwärtig Duftstofftücher verwendet oder auch Kunststoffbehälter, die den Duftstoff enthalten, welcher das Aroma durch Verdampfen verteilt. In beiden Fällen erfolgt die Abgabe des Aromas relativ unkontrolliert entweder durch die Wahl einer geeigneten Größe der Duftstofftücher oder indem der Öffnungsquerschnitt der Behälter geeignet dimensioniert wird. Beide Verfahren sind dadurch starken Schwankungen in Abhängigkeit von der Temperatur ausgesetzt. Außerdem ist ihre Anwendung nicht sehr nutzerfreundlich, und es fällt unnötigerweise Verbrauchsmaterial an. Die Verwendung von herkömmlichen Mikropumpen, die die eben genannten Nachteile nicht aufweisen, hat sich allerdings nicht durchgesetzt, da die Präzision, die diese bieten, bei der Aromatisierung nicht erforderlich und somit der Einsatz der teuren Präzisionspumpen nicht gerechtfertigt ist. Da die Duftessenzen jedoch sehr teuer sind, ist eine sparsame, bedarfsgesteuerte Anwendung aber durchaus erstrebenswert.

Der Erfindung liegt deshalb die Aufgabe zugrunde, eine preiswerte Vorrichtung zur Verfügung zustellen, welche über große Zeiträume eine Dosierung minimaler Stoffmengen gewährleistet. Gleichzeitig soll ein möglichst kompakter Aufbau der Vorrichtung durch Vermeidung beweglicher Teile realisiert werden, um dadurch Ausfälle, Wartung und Kosten zu reduzieren sowie die Lebensdauer der Vorrichtung zu erhöhen.

Diese Aufgabe wird erfindungsgemäß gelöst durch die Merkmale im kennzeichnenden Teil des Anspruchs 1 im Zusammenwirken mit den Merkmalen im Oberbegriff. Zweckmäßige Ausgestaltungen der Erfindung sind in den Unteransprüchen enthalten.

Danach ist funktionell zwischen dem Reservoir und dem Anwendungsraum eine Pumpenkammer angeordnet, deren Volumen durch die Tätigkeit der Mikromembranpumpe veränderbar ist. Pumpenkammer und Reservoir sind durch eine in Richtung auf die Pumpenkammer als Düse wirkende erste Öffnung und Pumpenkammer und Anwendungsraum durch eine in Richtung auf den Anwendungsraum als Düse wirkende zweite Öffnung verbunden. Die als Düse wirkende erste Öffnung ist so angeordnet, daß sie oberhalb des Befüllungsniveaus des Reservoirs liegt.

Die Erfindung soll nachstehend anhand des zumindest teilweise in den Fig. dargestellten Ausführungsbeispiels einer Vorrichtung zur Dosierung von Duftstoffen näher erläutert werden.

Es zeigen:
- Fig. 1: den prinzipiellen Aufbau des Mikrodosiersystems im Schnitt und
- Fig. 2: den prinzipiellen Aufbau eines piezo-keramischen Aktors.

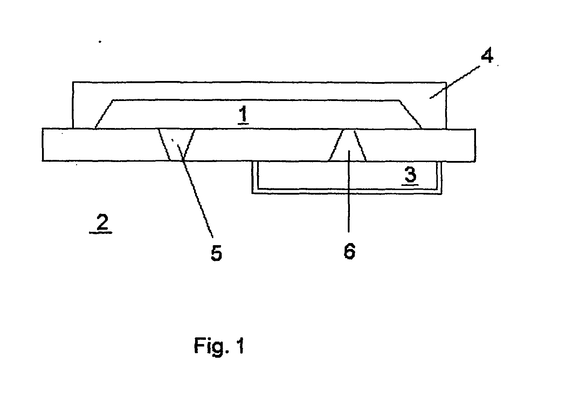

Die Vorrichtung nach Fig. 1 ist konzipiert für die Verteilung von Duftstoffen, z. B. für den Einsatz in Wäschetrocknern. Sie setzt sich zusammen aus einem Reservoir 3, in welchem sich der Duftstoff befindet, der Pumpenkammer 1, deren eine Wand von einer Mikromembranpumpe 4 gebildet wird, und dem Düse-Diffusor-System, welches die Pumpenkammer 1 mit dem Reservoir 3 und dem Anwendungsraum 2 verbindet. Der Anwendungsraum 2 kann offen oder auch ein geschlossener Raum sein. Im beschriebenen Ausführungsbeispiel grenzt das Reservoir 3 direkt an die Pumpenkammer 1. Es wird durch eine Trennwand, in welcher sich eine Öffnung 6 befindet, von der Pumpenkammer 1 getrennt. Diese in der Wand befindliche Öffnung 6 wirkt in der Richtung vom Reservoir 3 zur Pumpenkammer 1 als Düse, in der entgegengesetzten Richtung als Diffusor. Desgleichen grenzt die Pumpenkammer 1 direkt an den Anwendungsraum 2. In der Wand, die Anwendungsraum 2 und Pumpenkammer 1 trennt, befindet sich eine weitere Öffnung 5. Dabei wirkt diese Öffnung 5 in Richtung von der Pumpenkammer 1 zum Anwendungsraum 2 als Düse und in entgegengesetzter Richtung als Diffusor. Durch diese spezielle Gestaltung des Düse-Diffusor-Systems entsteht beim Pumpen an den beiden Düsen ein Druckunterschied, welcher bewirkt, daß ein im Reservoir 3 befindlicher Duftstoff in den Anwendungsraum 2 befördert wird.

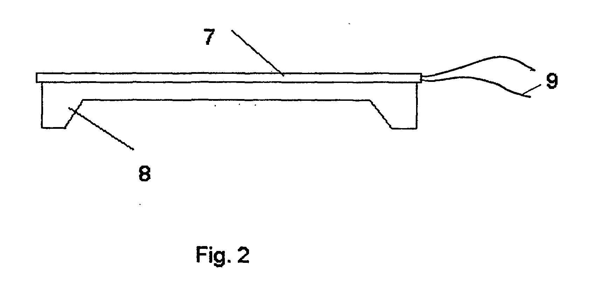

Die Mikromembranpumpe 4 wird aus einem piezo-keramischen Element 7 gebildet (Fig. 2), welches auf einem aus starrem Material bestehenden Träger 8 aufgebracht ist und durch Anlegung einer Spannung seine Längenausdehnung ändert, und somit ähnlich wie ein Bimetall bei Erwärmung wirkt. Die Wölbung, die durch die Längenausdehnung bewirkt wird, führt zu einer Verkleinerung bzw. Vergrößerung des Volumens der Pumpenkammer 1. An Stelle des piezo-keramischen Elements 7 mit Träger 8 könnte somit auch ein Bimetall-Aktor als Mikromembranpumpe 4 eingesetzt werden. Der Einsatz des piezo-keramischen Elements 7 bringt aber mehrere Vorteile mit sich. Für den Einsatz in Mikropumpen ist vor allem die hohe Frequenz, mit der Piezo-Elemente angesteuert werden können, in Verbindung mit dem Ausdehnungsverhalten, welches durch die angelegte Spannung äußerst genau gesteuert werden kann, von Bedeutung.

Die Vorrichtung nach der Erfindung befördert einen in einem Reservoir 3 befindlichen Duftstoff durch den Einsatz einer Mikromembranpumpe 4 über ein System aus Düse und Diffusor in festgelegten Dosen in einen Anwendungsraum 2. Man erreicht damit, daß der gasförmige Teil eines in einem Reservoir 3 als Flüssigkeit befindlichen Duftstoffes in geringen Mengen freigesetzt wird. Das wird durch den Einsatz eines Systems aus Düse (Öffnung 5) und Diffusor (Öffnung 6) in Kombination mit einer Mikromenbranpumpe 4 erreicht. Der Duftstoff befindet sich in einem Reservoir 3, welches über die Öffnung 6 mit einer Pumpenkammer 1 in Verbindung steht, welche wiederum mit dem Anwendungsraum 2 verbunden ist. Die Mikromenbranpumpe 4 bewirkt, daß sich Volumen und Druck in der Pumpenkammer 1 periodisch verändern, verkleinern bzw. vergrößern. Durch Volumenvergrößerung, d. h. Druckverminderung wird durch den Diffusor (Öffnung 6) aus dem Reservoir 3 ein Teil des in gasförmiger Form vorliegenden Duftstoffes in die Pumpenkammer 1 gesaugt, da infolge der Dimensionierung von Düse (Öffnung 5) und Diffusor (Öffnung 6) die Sogwirkung am Diffusor 6 größer ist als an der Düse 5. Die Folge davon ist, daß zwar aus dem Reservoir 3 Stoff in die Pumpenkammer 1 gelangt, jedoch kaum Gas aus dem Anwendungsraum 2 in die Pumpenkammer 1 gelangt. Wird das Volumen der Pumpenkammer 1 anschließend verkleinert (der Druck erhöht), so gelangt durch die spezielle Konstruktion der Düse bzw. des Diffusors der in der Pumpenkammer 1 befindliche Stoff in den Anwendungsraum, nicht oder nur in geringerem Maße in das Reservoir 3. Bei periodischer Änderung des Volumens der Pumpenkammer 1 wird so ein im Reservoir 3 befindlicher Stoff im Laufe der Zeit langsam und in vorgegebenen, minimalen Dosen in den Anwendungsraum 2 befördert.

Ein möglicher Einsatzbereich des Mikro-Dosierungsystems bietet sich beispielsweise beim Trockenvorgang im Wäschetrockner an. Das Mikrodosiersystem kann dabei im Trockner integriert werden. Da von dem Duftstoff je Trockenvorgang nur äußerst geringe Mengen benötigt werden, reicht ein entsprechend kleines Reservoir 3 über einen langen Zeitraum, für den man als Anhaltspunkt von der mittleren Lebensdauer des Trockners ausgehen kann.

Der Einsatz dieses Mikrodosiersystems mit geeignet dimensionierten Düsen zusammen mit dem Reservoir 3 besitzt keine beweglichen Teile, und ist somit praktisch wartungsfrei, klein und kostengünstig.

Die Erfindung ist nicht beschränkt auf das hier dargestellte Ausführungsbeispiel. Vielmehr ist es möglich, durch Kombination und Modifikation der genannten Mittel und Merkmale weitere Ausführungsvarianten zu realisieren, ohne den Rahmen der Erfindung zu verlassen.

## Patentansprüche

1. Dosiervorrichtung zur Förderung geringer Stoffmengen in einen Anwendungsraum (2) mit einem Reservoir (3) und einer Mikromembranpumpe (4), wobei
- zwischen Reservoir (3) und Anwendungsraum (2) eine Pumpenkammer (1) angeordnet ist, deren Volumen durch die Tätigkeit der Mikromembranpumpe (4) veränderbar ist,
- Pumpenkammer (1) und Reservoir (3) durch eine erste Öffnung (6) und
- Pumpenkammer (1) und Anwendungsraum (2) durch eine in Richtung auf den Anwendungsraum (2) als Düse wirkende zweite Öffnung (5) verbunden sind,
**dadurch gekennzeichnet, dass**
die erste Öffnung (6) in Richtung auf die Pumpenkammer (1) als Düse wirkt und zur Förderung eines gasförmigen Teils einer Flüssigkeit im Reservoir (3) so angeordnet ist, dass sie oberhalb eines Befüllungsniveaus der Flüssigkeit liegt.

2. Dosiervorrichtung nach Anspruch 1,
**dadurch gekennzeichnet, dass**
Pumpenkammer (1) und Reservoir (3) eine gemeinsame Begrenzungsfläche besitzen.

3. Dosiervorrichtung nach Anspruch 1 oder 2,
**dadurch gekennzeichnet, dass**
Pumpenkammer (1) und Anwendungsraum (2) eine gemeinsame Begrenzungsfläche besitzen.

4. Dosiervorrichtung nach einem der Ansprüche 1 bis 3,
**dadurch gekennzeichnet, dass**
die Mikromembranpumpe (4) eine Begrenzungsfläche der Pumpenkammer (1) bildet.

5. Dosiervorrichtung nach einem der Ansprüche 1 bis 4,
**dadurch gekennzeichnet, dass**
die Mikromembranpumpe (4) die Begrenzungsfläche zwischen Pumpenkammer (1) und Reservoir (3) bildet.

6. Dosiervorrichtung nach einem der Ansprüche 1 bis 5,
**dadurch gekennzeichnet , dass**
die Mikromenbranpumpe (4) als piezokeramischer Aktor ausgebildet ist.

7. Dosiervorrichtung nach einem der Ansprüche 1 bis 5,
**dadurch gekennzeichnet, dass**
die Mikromenbranpumpe (4) als Bimetall-Aktor ausgebildet is

## Claims

1. Metering device for conveying small substance quantities into a use space (2), with a reservoir (3) and a micro-diaphragm pump (4), wherein
- a pump chamber (1) is arranged between reservoir (3) and use space (2) and has a volume variable by the actuation of the micro-diaphragm pump (4),
- pump chamber (1) and reservoir (3) are connected by a first opening (6) and
- pump chamber (1) and use space (2) are connected by a second opening (5) acting in the direction of the use space (2) as a nozzle,
**characterised in that** the first opening (6) acts in the direction of the pump chamber (1) as a nozzle and is so arranged for conveying a gaseous component of a liquid into the reservoir (3) that it is disposed above a filling level of the liquid.

2. Metering device according to claim 1, **characterised in that** the pump chamber (1) and the reservoir (3) have a common boundary surface.

3. Metering device according to claim 1 or 2, **characterised in that** the pump chamber (1) and the use space (2) have a common boundary surface.

4. Metering device according to one of claims 1 to 3, **characterised in that** the micro-diaphragm pump (4) forms a boundary surface of the pump chamber (1).

5. Metering device according to one of claims 1 to 4, **characterised in that** the micro-diaphragm pump (4) forms the boundary surface between pump chamber (1) and reservoir (3).

6. Metering device according to one of claims 1 to 5, **characterised in that** the micro-diaphragm pump (4) is constructed as a piezo-ceramic actuator.

7. Metering device according to one of claims 1 to 5, **characterised in that** the micro-diaphragm pump (4) is constructed as a bimetallic actuator.

## Revendications

1. Dispositif de dosage pour acheminer des petites quantités de substance dans un espace d'utilisation (2), qui présente un réservoir (3) et une micropompe (4) à membrane,
- une chambre de pompe (1) dont le volume peut être modifié sous l'action de la micropompe (4) à membrane étant agencée entre le réservoir (3) et l'espace d'utilisation (2),
- la chambre de pompe (1) et le réservoir (3) étant reliés par une première ouverture (6) et
- la chambre de pompe (1) et l'espace d'utilisation (2) étant reliés par l'intermédiaire d'une deuxième ouverture (5) qui agit comme ajutage en direction de l'espace d'utilisation (2),
**caractérisé en ce que**
la première ouverture (6) agit comme ajutage en direction de la chambre de pompe (1) et est agencée de telle sorte qu'elle est située au-dessus d'un niveau de remplissage de liquide pour l'acheminement de la partie gazeuse d'un liquide dans le réservoir (3).

2. Dispositif de dosage selon la revendication 1,
**caractérisé en ce que**
la chambre de pompe (1) et le réservoir (3) présentent une surface frontière commune.

3. Dispositif de dosage selon les revendications 1 ou 2,
**caractérisé en ce que**
la chambre de pompe (1) et l'espace d'utilisation (2) présentent une surface frontière commune.

4. Dispositif de dosage selon l'une des revendications 1 à 3,
**caractérisé en ce que**
la micropompe (4) à membrane forme une surface frontière de la chambre de pompe (1).

5. Dispositif de dosage selon l'une des revendications 1 à 4,
**caractérisé en ce que**
la micropompe (4) à membrane forme la surface frontière entre la chambre de pompe (1) et le réservoir (3).

6. Dispositif de dosage selon l'une des revendications 1 à 5,
**caractérisé en ce que**
la micropompe (4) à membrane est configurée comme actionneur piézocéramique.

7. Dispositif de dosage selon l'une des revendications 1 à 5,
**caractérisé en ce que**
la micropompe (4) à membrane est configurée comme actionneur à bimétal.
